Europäisches Patentamt

**European Patent Office**

Office européen des brevets

(11) Publication number: **0 029 640**
**A1**

(12) **EUROPEAN PATENT APPLICATION**

(21) Application number: **80302286.2**

(22) Date of filing: **04.07.80**

(51) Int. Cl.³: **A 61 L 2/20**
**A 23 B 5/00, A 23 L 3/34**
**A 23 B 7/144, A 23 B 9/00**

(30) Priority: **23.11.79 GB 7940583**

(43) Date of publication of application:
**03.06.81 Bulletin 81/22**

(84) Designated Contracting States:
**BE DE FR GB IT NL**

(71) Applicant: **PAULS & WHITES INTERNATIONAL (GB) LIMITED**
**Springfield Road**
**Burnham-on-Crouch Essex, CMO 8TA(GB)**

(72) Inventor: **Field, Jeffrey Alan**
**11 Burns Close**
**Maldon, Essex(GB)**

(72) Inventor: **Hammond, William Charles**
**39 St.Nicholas Road**
**Tillingham, Essex(GB)**

(74) Representative: **BAYLISS, Geoffrey Cyril et al,**
**BOULT, WADE & TENNANT 27 Furnival Street**
**London EC4A 1PQ(GB)**

(54) Improvements in or relating to fumigation apparatus.

(57) The disclosure relates to fumigation apparatus particularly for fumigating eggs comprising a cabinet (10) to receive a supply of eggs or any other matter to be fumigated a chamber (28) extending across the top of the cabinet and two ports (23,24) spaced across the top of the cabinet communicating the chamber with the interior of the cabinet. One of the ports (23) contains a motor driven fan (25) for circulating a fumigating atmosphere in the path down one side of the cabinet, up the other side, into the chamber, and then back into the cabinet to wash over the eggs stored in the cabinet and destroy the bacteria thereon. An air inlet duct (40) is mounted at one end of the chamber (28) and an air exhaust duct (40) is mounted at the other end of the chamber (28). Each inlet and exhaust duct contains a barrier formed by a liquid reservoir (45) and a baffle plate (48) extending downwardly into the top of the reservoir from the top of the duct to form a U-shaped passage through the reservoir which can be opened and closed by raising and lowering the level of liquid to the reservoir by means of a separate header tank (50) pivotally mounted on the outside of the duct. The inlet and exhaust ducts can thus be closed to prevent escape of fumigant from the cabinet when the fumigation process is being performed.

EP 0 029 640 A1

./...

FIG. 1.

FIG. 4.

FIG. 6.

"IMPROVEMENTS IN OR RELATING TO FUMIGATION APPARATUS"

This invention relates to fumigation apparatus for fumigation of hatching eggs and any other matter which it is required to fumigate to kill surface bacteria such as plants.

Fumigation of eggs with formaldehyde gas is necessary in order to kill bacteria which may be present on the surface of the eggs. This is best done as soon as possible after an egg has been laid.

When an egg is laid, it is at the temperature of the body of the hen but rapidly cools. The egg shell is full of pores through which it is possible for bacteria to enter. If the egg is laid in a dirty nest box or on the floor litter of a poultry house bacteria will enter the shell through the pores while the egg is still warm before it has contracted slightly and the pores have closed up.

When the egg is incubated, the pores open up again in the warm, humid environment of the incubator. The pores in the shell are necessary because they enable the developing embryo of the chick to take in oxygen and expel carbon dioxide.

Formaldehyde gas will kill most germs and
bacteria but it is vital that the gassing is done as
quickly as possible after the egg has been laid in
order to kill these germs and bacteria while they are
still present on the surface of the egg shell or at
worst have only just got under the surface of the shell.
It has been the practice for many years to collect
eggs from farms once or twice a day to fumigate the eggs
at the hatchery. Sometimes eggs are held for up to seven
days in a cold room and are fumigated in a large fumigation
chamber either immediately before or at best twenty-
four hours before placing them in an incubator. In
many cases the fumigation is of very little use at this
stage because the bacteria have had plenty of time to
get into the egg and the gas will not kill them. An
incubator is then an ideal breeding ground for bacteria and
they multiply rapidly within the egg which eventually
results in the death of the developing embryo.

When the eggs are gassed they are placed in a
fumigation chamber where the temperature is raised
to about 90°F. and the relative humidity is about
85%. This tends to open the pores of the eggs and
allows the gas to penetrate.

If the eggs are fumigated at the farm, the chances
are greater of killing any bacteria present and therefore
ensuring that the maximum number of embryos survive to become live chicks.

The world poultry industry has been aware of the need to fumigate eggs as early as possible but until recently the capital cost of installing a fumigation chamber at each farm has outweighed the advantages of early fumigation. The usual fumigation chamber at a commercial hatchery is of brick and cement construction with refrigeration type doors and expensive ventilation, heating and humidification equipment. Depending on the capacity of the hatchery it can have up to twenty farms supplying it with hatching eggs. To build an efficient fumigation chamber on each farm would therefore be very costly.

The fumigation chamber described below has been designed to do all of the things that a large brick built chamber will do but at a fraction of the capital cost and at a very low operating cost. Several such fumigation chambers for 'on-farm' gassing have been built before this invention but because of their design they have either failed to fumigate all of the eggs properly or have leaked gas into the egg room or both.

Formaldehyde gas can cause irritation to the skin, eyes and respiritory tract and has an unpleaseant smell. Health and Safety Regulations in many countries make it impossible to operate egg fumigation chambers which may be a health hazard to the operators.   The fumigation cabinet described has been so designed that it is completely safe to use while being extremely efficient in fumigation of hatching eggs.

Where other matters such as vegetable matter is to be fumigated, other fumigants may be used but it is invariably the case that such fumigants are either noxious or have unpleasant side effects and so it is important to prevent release of such fumigants to the environment in  which the operator works.

The invention provides fumigation apparatus comprising, a gas tight cabinet to receive the matter to be fumigated, at least one sealable opening in the cabinet through which eggs to be fumigated can be placed in or removed from the cabinet, means to heat the cabinet, means to circulate a fumigant in the cabinet, an exhaust outlet for fumigant from the cabinet, an air inlet into the cabinet and means for closing both the inlet and outlet to the cabinet comprising, in both the inlet and outlet, U-shaped passageways and means to supply liquid to the bottom of each passageway and means to raise the level of liquid in the passageway to a level at which the two sides of the U are cut off from each other to close the passageway and to lower the liquid level to a level in which the two sides of the U are in communication to open the passageway.

The following is a description of some specific embodiments of the invention, reference being made to the accompanying drawings in which:-

Figure 1 is front perspective of a fumigation cabinet;

Figure 2 is a side view of the cabinet;

Figures 3 to 6 illustrate air inlet and exhaust outlet ducting arrangements for the cabinet;

Figure 7 illustrates a heater and thermometer tray assembly for the cabinet;

Figure 8 illustrates a moisture tank assembly for the cabinet;

Figure 9 illustrates a header tank for the cabinet; and

Figure 10 illustrates a trolley for supporting trays of hatching eggs for use with the cabinet.

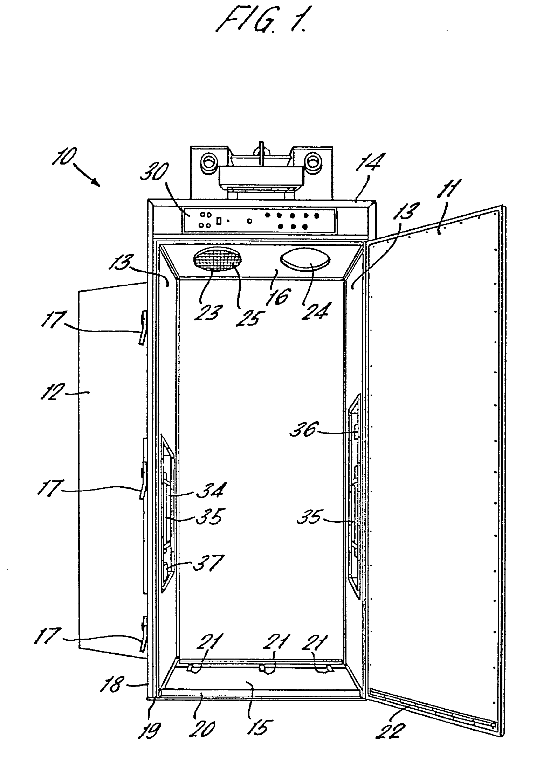

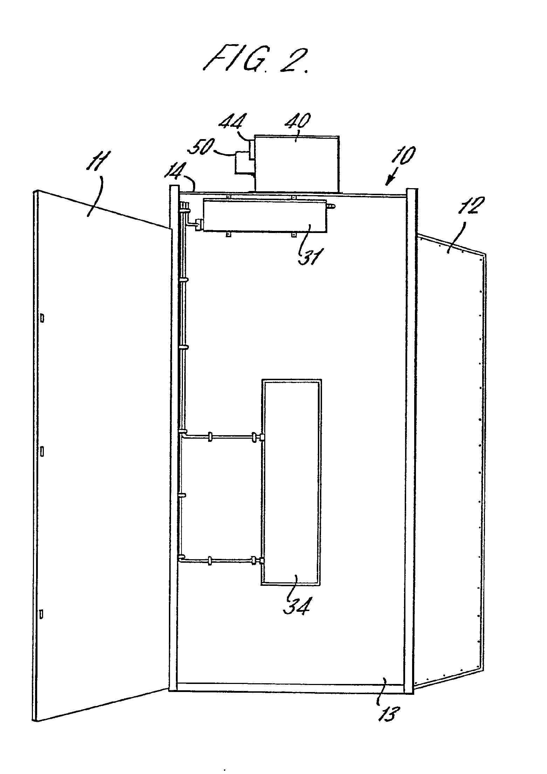

Referring now to Figures 1 and 2 of the drawings, there is shown a fumigation cabinet indicated generally at 10 which is in the form of an upright rectangular box having front and rear doors 11 and 12 respectively, sides 13, a roof 14 and a base 15.

The cabinet is built of a material known as 'farm-board' which is impervious to formaldehyde gas. Each door is made of plywood fastened to a softwood frame and the inner surface of the door is clad with galvanised steel sheet. The cabinet has a false ceiling 16 which is also made of 'farm-board'.

The floor of the cabinet is made of steel plate of a thickness of three-sixteenths of an inch or one-eighth of an inch and is electro-galvanised to prevent corrosion.

The whole cabinet is sealed at all the joints with a mastic sealer as well as being nailed and glued when constructed. The framework on the outside is made of hardwood. The 'farm-board' panels which make up the sides, back, roof and false ceiling are fastened to the framework. Each door is hinged at one side to the framework and fastened at the other side with three handles 17. The handles 17 are of such design that they pull the door in towards the framework. The door is not recessed but is on the outside of the framework.

A groove 18 is cut into the framework at each side of the door and at the top of the door. A neoprene rubber strip 19 is inserted and glued into this groove and stands proud of the framework by approximately a quarter of an inch. When the door is closed, it is pressed against this strip, compressing it and thus forming an efficient seal at the sides and top of the door.

The cabinet is of such dimensions that it is capable of housing a trolley which is laden with trays of eggs. When the trolley has been pushed into the cabinet, a length of angled steel 20 is fitted onto the steel floor at the entrance to the cabinet. This clips into three brackets 21 which are welded to the steel floor. The cabinet frame is recessed slightly to allow a length of angled steel which is slightly longer than the interior width of the cabinet to fit across the base of the cabinet.

The door has a strip of neoprene rubber 22 fastened across its width about half an inch from the bottom of the door on the inner lining of the door. When the door is closed, this neoprene strip is compressed against the angled steel and therefore provides a gas-tight seal.

The provision of a door at the back as well as a door at the front allows a trolley full of eggs to be removed into a sterile room at the other side of the fumigation cabinet. The door is sealed in exactly the same manner as described previously. It is thought to be an improvement to have a second door and to position the cabinet in the egg room of a poultry house or farm egg store so that the room can be divided and the cabinet to be fitted into a dividing wall. Eggs are then placed into the cabinet through the front door, from the room which is considered to be the 'dirty' area. When the gassing cycle has finished, the eggs are removed through the rear door into the 'clean' area. By not removing the fumigated eggs via the front door there is less risk of the eggs being contaminated after fumigation by air-borne bacteria. The cabinet can however be provided with a single door only.

The false ceiling 16 of the cabinet has in it two holes of equal diameter. Into the left hand hole is fitted a fan and motor unit 25. This motor 23, 24 can turn in a clockwise or anti-clockwise direction and the impellor is so designed that it can push air or pull air. The reason for this is described later.

A trolley 26 for holding a supply of eggs to be fumigated is shown in Figure 10 and is constructed of square steel tubing and angled steel, which is hot dipped galvanised after construction. The trolley is mounted on two fixed and two swivel castors 27 or alternatively four swivel castors. The trolley is so designed as to be capable of holding plastic trays each 35 inches long by 12 inches wide by 2 inches high. The trays slide onto angled steel runners which are fastened within the framework of the trolley. Each plastic tray is capable of holding in individual pockets, 132 chicken eggs. A similar tray of the same dimensions is capable of holding in individual pockets, 100 turkey eggs or 100 duck eggs.

Three types of trollies are available. Each trolley is designed to hold two stacks of trays and is therefore approximately 28 inches wide by 37 inches long. A trolley holding two stacks of fifteen trays (3960 chicken eggs), two stacks of eighteen trays (4752 chicken eggs), two stacks of sixteen trays (3200 turkey or duck eggs) is available. The cabinet is so constructed that it will accept all three trollies. The trollies are identical in length and breadth but vary in height.

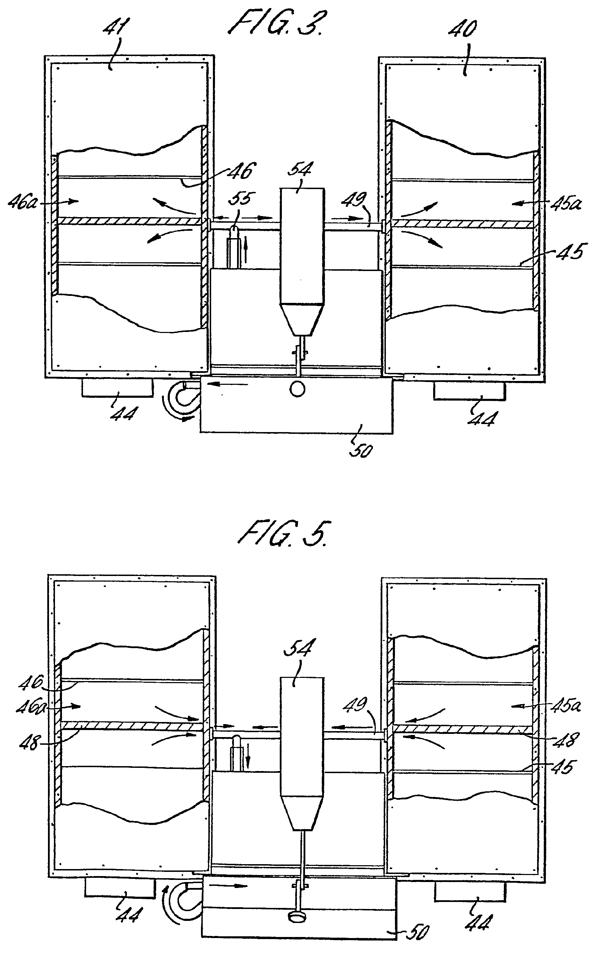

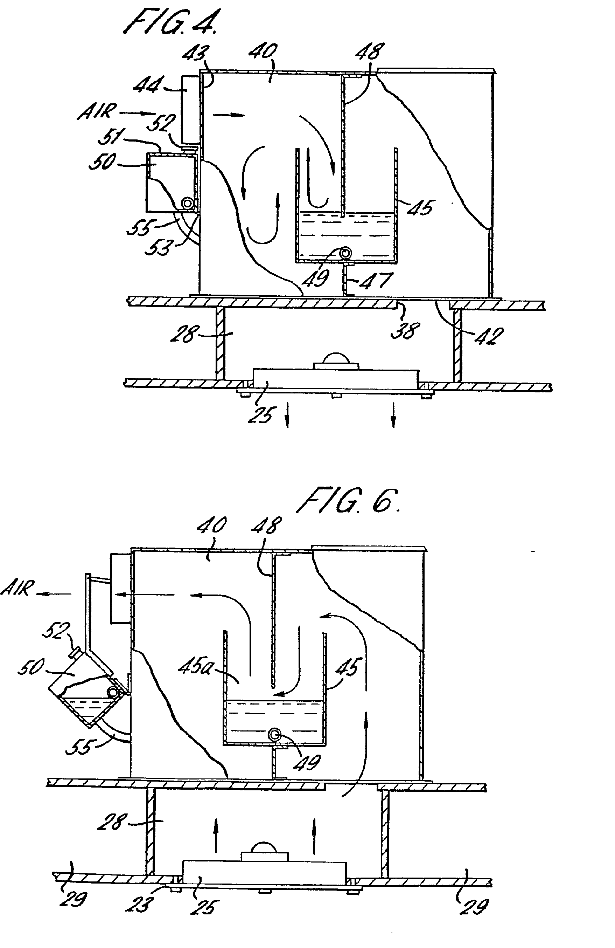

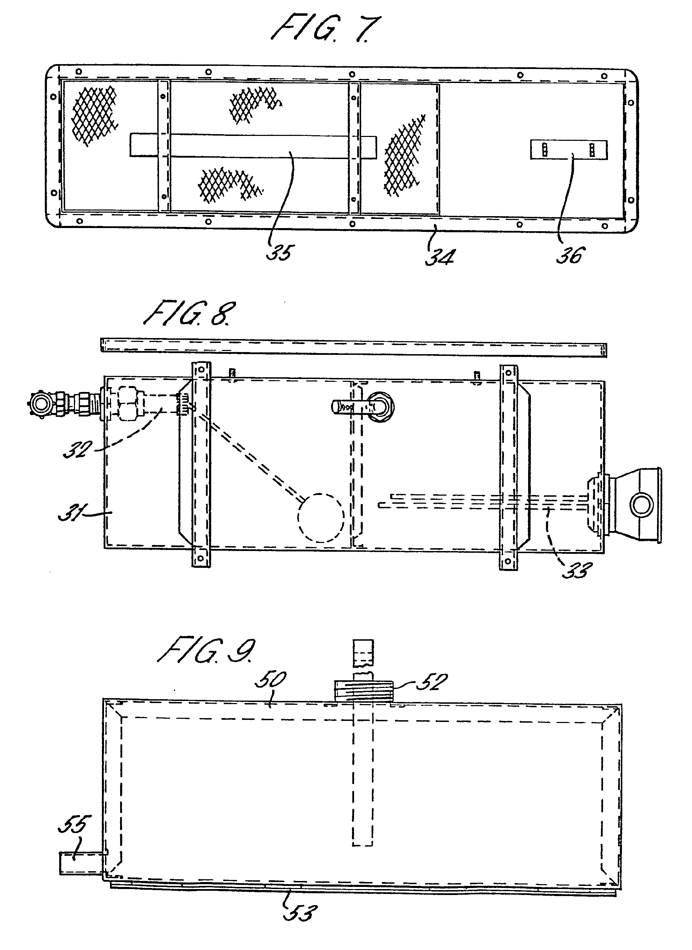

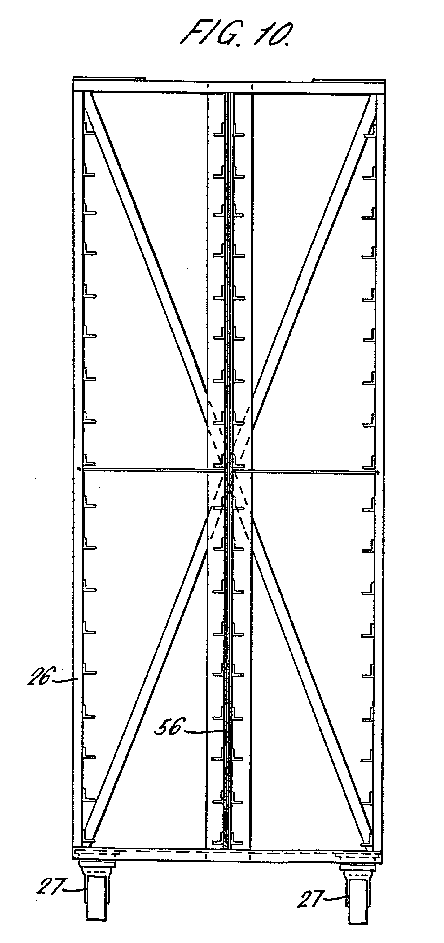

Between the roof of the cabinet and the false ceiling is a space which is divided into three separate chambers as can be seen in Figures 4 and 6. The centre chamber 28 is sealed in order that gas may not escape but is open to the interior of the cabinet by the two large diameter holes 23, 24, one of which has the fan 25 mounted over the hole. The outer chambers 29 contain the control panel 30 at the front, and if it is a cabinet with front and rear doors, has a second electrical panel contained within the rear chamber.

Mounted on the outside of the cabinet on the right-hand side near the top is a stainless steel tank 31 containing a ball valve 32 and an immersion heating element 33 as shown in Figure 8. When the fumigation cabinet is going through its gassing cycle, the immersion heater switches on automatically, heats the water and creates steam. The steam enters the centre chamber 28, between the cabinet roof and the false ceiling, through a copper tube (not shown). This tube is of half an inch diameter and is connected through the side of the stainless steel tank above the water level. It enters the centre chamber through a hole in the side of the cabinet and runs at a slight incline up into the centre chamber. The end of the tube which protrudes into the cabinet is capped and a series of small holes are drilled in the wall of the tube on the uppermost portion. Steam can escape through these small holes into the centre chamber. By having the tube running up an incline, any steam which condenses in the tube will not cause dripping onto the false ceiling. The water will flow back to the tank. The purpose of this steam vessel is twofold. It provides the necessary humidity within the fumigation cabinet and also assists with the heating of the cabinet. As the steam vessel is at head height for the average human, it is covered by a suitable guard to prevent anyone accidentally coming into contact with the hot metal of the vessel.

Inside the cabinet there are recesses in both of the side walls into which are fitted mild steel cadmium plated 'trays' 34 (see Figure 7). These recessed sections each contain an electric heating element 35, each being 650 watts. The elements are suitably guarded to prevent anyone from touching a hot element or being electrically shocked by touching a 'live' terminal. The recesses are long enough to enable the air and gas which is being driven by the fan, to pick up the heat from the heating element without interrupting the air-flow and causing currents of air to 'eddy'.

Into the top of one of the recesses is fitted an electrical contact thermometer 36. This thermometer is held in place by two spring clips through which a current of electricity can pass. When the desired temperature has been reached, the mercury in the thermometer completes the circuit by bridging the two clips, which are connected to electrical wires to a relay in the control panel. The action of the mercury 'making' and 'breaking' will cause both heating elements to be switched on and off. This controls the heat in the fumigation cabinet to a certain accuracy. The thermometer is of the fixed contact type which is set at 85°F., but this can be of whatever temperature is requested by a client. As long as the temperature of the eggs does not exceed 100°F. for more than about 30 minutes, the temperature setting is not critical.

The heaters are switched on and off according to the reading of the thermometer but is is possible for the heat to over-ride this fixed temperature a little due to the heat given off by the steam tube.

The gas is produced by one of two methods, according to the customers' requirements. The usual method of releasing gas in large fumigation chambers is by adding a 40% formalin solution to crystals of potassium permanganate.   The chemical reaction causes the liquid to heat rapidly to about $450^{o}$C. and gas is released. This method is used in the fumigation cabinet.   Potassium permanganate crystals are placed in a stainless steel container 37.   The container is placed into the left-hand recess in the cabinet.   A stainless steel tube leads from a funnel (not shown) on the outside of the cabinet to a point inside just above the container.   The method is to place a measured amount of crystals into the container, to place the container in position in the left-hand recess, to wheel in the trolley full of eggs to be fumigated, close and fasten the door.   After pre-heating the cabinet for ten minutes, during which time the steam vessel is in operation, the formalin solution is poured into the funnel and a stainless steel spring loaded cap is "locked" onto the top of the funnel to stop any escape of gas.   The machine is then switched on to its automatic cycle.

An alternative system is available, which avoids the mecessity of measuring out the correct amount of crystals of potassium permanganate and formalin solution for each fumigation operation.   For the alternative system an aluminium vessel containing a heating element is fitted into the left-hand recess in the inside of the cabinet. Into this vessel a measured amount of crystals of paraformaldehyde are placed. After the eggs are put into the cabinet and the door is closed, the machine is switched on and the heating element in the aluminium vessel is automatically switched on. The heat causes the crystals to release formaldehyde gas. The element is controlled by a timer which switches it off after all the crystals have turned into gas.

Although the cabinet is completely sealed, it must have an outlet through which to disperse the gas when the fumigation cycle is completed. It must also have an air inlet to admit fresh air to the cabinet in order to replace the gas and air mixture which is being expelled. The construction of the air inlet and exhaust outlet will now be described with reference to Figures 3 to 6.

In the roof of the cabinet are two oblong holes 38 (only one of which can be seen) in communication with the central chamber 28 above the false ceiling of the cabinet, the holes being positioned adjacent the large diameter holes 23, 24 in the false ceiling respectively. Mounted over these oblong holes on the ceiling are two stainless steel ducts 40, 41 which are oblong in shape and each duct has a lid which is fastened down and is made air-tight by a neoprene rubber seal (not shown) around the top edge. The end wall of each duct remote from the aperture 42 in the base has an opening 43 formed with a peripheral flange 44 for connection to a further conduit.

Mid-way along each duct 40,41 there is a transversely extending open topped tank 45, 46 which extends across the full width of the tank and is supported on a wall 47 also extending across the full width of the tank. A baffle plate 48 extends downwardly from the top of each duct stopping short of the bottom of the duct to define U-shaped passages 45a, 46a through the tanks underneath the baffle plates. The tanks 45, 46 contain a liquid, preferably light oil, which can be raised above the lower edge of the baffle plate to close off the passageway between one side of the baffle and the other or lowered below the baffle plate to open the passageway from one side of the baffle to the other as illustrated in Figures 4 and 6 respectively.

The two tanks 45,46 are connected together by a pipe 49 so that as the level of the liquid is raised or lowered, the levels will always be identical.

In order to raise and lower the liquid level it is necessary to have a tank 50 to drain into or fill up from. The third tank is a closed vessel 50 which has a filler hole 51 in the top with a screw cap 52 and has an air vent (not shown). It is situated forward of the other tanks and is hinged at 53 along the base at the rear so that it can either be upright or tilted forward by an actuator 54. It is connected to the other tanks by a flexible pipe 55 which has a junction with the pipe that connects the two main tanks.

If this vessel is tilted forwards to an angle of 45 degrees, the greater portion of it is below the level of the main tanks and the liquid will draim from those tanks into this holding vessel. When moved into the upright position, the liquid will flow back into the main tanks and the liquid level will rise sufficiently to close off the gap between the surface of the liquid and the bottom of the baffle plates. This liquid reservoir is tilted forwards and backwards by an electrically propelled piston or actuator.

The control panel 30 for the fumigation cabinet is situated in the front cavity between the false ceiling and the roof and has a facia panel which has various switches and pilot lamps to monitor the fumigation cycle.

Apart from a mains switch, there are two fuses for the protection of the instrument circuit and heater circuit respectively. Each fuse has a small pilot lamp situated below the fuse. Should a fuse 'blow' the pilot lamp will light.

The mains switch also has a pilot lamp to indicate that the power is switched on.

There is a cycle button which must be depressed in order to start the fumigation cycle.

There is a two-way switch to put the machine on to preheat or cycle.

Additionally there are several pilot lamps which indicate 'cycle on', 'heaters','gassing', 'exhaust', 'cycle-off' and 'preheat'.

The trollies which go into the fumigation cabinet, have a divider 56 between the two columns of trays. This is made of galvanised sheet metal and is permanently fixed within the framework of the trolley. There is a flap made of rubber sheeting, fixed to the ceiling of the cabinet. When the trolley is in the cabinet, this flap is in line with the sheet metal divider in the trolley, It therefore, together with the metal divider, separates the left-hand and right-hand portions of the cabinet, with the exception of the space underneath the trolley frame to provide with the chamber at the top of the cabinet, a path around which the fumigant is circulated by the fan to act on the eggs on the trolley.

The fumigation operation is as follows:
Potassium permanganate crystals or Paraformaldehyde crystals are placed in the receptacle provided inside the cabinet. The type of crystals used depends on the type of fumigation cabinet, as has been previously described. The trolley laden with trays of eggs is then pushed into the cabinet.   The piece of
angled steel is placed in position on the floor of the
cabinet in the doorway and the door is closed and
fastened.   The mains switch is put to the 'on'
position and the two-way switch to  the 'preheat'
position.   This starts the fan motor and energises
the heaters and also the immersion heater in the steam
vessel.   The fan blows air downwards through the
left-hand column of trays, underneath the trolley, upwards through the right-hand column of trays, through the
hole in the right-hand portion of the false ceiling,
across the chamber above the ceiling and back to the
fan.   The air is therefore being forced around the
cabinet in an anticlockwise direction.   On the way
round, the air picks up warmth from the heaters and
steam from the steam-pipe.   After a few minutes
the cabinet should be sufficiently heated for the
heater pilot lamp to go off, indicating  that the
desired temperature has been reached.   The two-way
switch is now put to the 'cycle' position and the
cycle button depressed.   Everything will now be done
automatically.   The heater in the fumigation vessel
will now be energised and gas will be released from
the crystals  of paraformaldehyde.

        If the machine is built for the other type of fumigant, then the formalin should be poured in through the funnel and the spring loaded cap replaced before pressing the 'cycle' button.

The fan will continue to drive the gas around the cabinet for a period of twenty minutes, during which time the heaters will work intermittently in order to keep the desired temperature and the steam vessel will operate continuously.

After the twenty minutes gassing cycle, the fan will stop, the heaters will go off and the steam vessel will cease operation. The gas is then allowed to remain static for a period of ten minutes. Following this, the fan motor starts again but this time in the opposite direction so that it is pulling instead of pushing the gas. The actuator on the top of the cabinet will move the holding vessel forwards and oil will flow into it from the main tanks. This will allow the gas to be forced out by the fan, which at the same time will draw in fresh air to replace the gas.

Twenty minutes is the time allowed for the exhaust cycle. When this time has elapsed, the fan will stop and the actuator will move the holding vessel into the upright position in order to refill the main tanks with oil in readiness for the next fumigation cycle.

The door can now be opened and the trolley full of eggs removed.

During the fumigation cycle the pilot lamps on the facia of the control panel will indicate what is happening in the machine, i.e. gassing, heaters on, exhausting, cycle finished etc.

On models which have a door at the rear, a monitor panel is fitted into the rear chamber between the ceiling and the roof and pilot lamps on the facia of this panel will indicate to the person who is to remove the eggs into the 'clean' room, what is happening and when it is safe to open the rear door.

Although the cabinet is absolutely gas-tight, there may still be a smell of gas when the eggs are removed. This is due to gas which has entered through the pores of the eggs, escaping slowly into the air. However, the concentration is so low that it is completely safe and is not a health hazard. The smell of gas is less obvious from one trolley full of eggs after removal from a small fumigation chamber than it is from several trolley loads of eggs which have been removed from a large fumigation room in a commercial hatchery.

The cabinet described above is intended particularly for the fumigation of hatching eggs using a formaldehyde fumigant. The cabinet is however equally applicable to fumigation of other material including vegetable matter and it will be understood that the fumigant used and the operating conditions including temperature and humidity are selected to suit the matter to be fumigated. For example the cabinet is particularly suitable for fumigation of small plants or seedlings for which a nicotine fumigant is used. Nicotine is supplied in liquid form to the fumigation vessel of the cabinet to produce the fumigating atmosphere. The internal temperature of the cabinet is maintained at a lower level than that used for fumigating eggs and the steam supply system for raising the humidity in the cabinet is not used. A trolley substantially the same as that used for carrying the eggs is used for supporting the plants/seedlings except that the trolley is equipped with performated shelves to support the plants/seedlings and allow the flow of fumigatory gas to pass through the shelves and over the plants/seedlings stored thereon.

Claims:-

1.    A fumigation apparatus comprising, a gas tight
cabinet (10) for the matter to be fumigated, at least
one sealable opening in the cabinet through which matter
to be fumigated can be placed in or removed from the
cabinet, means (35) to heat the cabinet, means (25)
to circulate a fumigant in the cabinet, an exhaust
outlet (40) for fumigant from the cabinet, an air
inlet (41) into the cabinet and means (45) for closing both
the inlet and outlet to the cabinet, characterised in
that both the inlet and outlet contain U-shaped
passageways (45a,46a) and means are provided to supply liquid
(50,54,55) to the bottom of each passageway and to
raise the level of liquid in the passageway to a
level at which the two sides of the U are cut off
from each other to close the passageway and to lower
the liquid level to a level in which the two sides of
the U are in communication to open the passageway.

2.    An apparatus as claimed in claim 1 characterised
in that the air inlet (41) and exhaust outlet (40)
each comprise a duct (40,41) with an open topped tank
(45, 46) mounted on the bottom of the duct and
extending across the duct and a baffle (48) mounted at the top of the duct and extending downwardly into the tank (45,46) but stopping short of the bottom of the tank to form the U-shaped passageway (45a, 46a) through the tank, means (50,54,55) being provided for supplying liquid to and drawing liquid from the tank to control the liquid level in the tank in relation to the bottom of the baffle so that the liquid level may be raised above the bottom of the baffle to close off the passageway and therefore the duct and to lower the liquid level below the bottom of the baffle to open the passageway and therefore the duct.

3. An apparatus as claimed in claim 2 characterised in that the two ducts are located side-by-side with the tanks (45,46) and baffles (48) level with each other and the means for raising and lowering the liquid levels in the conduit comprises a single reservoir (50) having a conduit (55) connection to the tanks and means (54) to raise and lower the reservoir to raise and lower the liquid levels in the tanks (45,46) in relation to the baffles (48):

4. An apparatus as claimed in claim 3 characterised in that the tanks (45,46) are connected by a conduit (49) to ensure a common liquid level in the two tanks.

5. An apparatus as claimed in any of the preceding claims characterised in that air inlet and exhaust outlets (40,41) extend from inlet and outlet apertures (38) spaced apart across the top wall (14) of the cabinet and the cabinet has a false ceiling (16) spaced below the top wall (14) of the cabinet with two openings (23,24) spaced apart across the false ceiling and fan means (25) are provided for causing a circulation of the fumigant around the cabinet and through the false ceiving when the inlet and outlets are closed during the fumigation stage,and, when the inlet and outlets are open,for discharging fumigant and drawing in fresh air at the end of the fumigation process.

6. An apparatus as claimed in claim 5 characterised in that the fan means (25) comprise a single fan (25) located in the aperture (23) in the false ceiling (16) adjacent the opening (38) in communication with the exhaust outlet (40) to drive out the fumigant from the cabinet through the exhaust outlet (40) and draw in fresh air into the cabinet (10) when the inlet (41,40) and outlet are open and to circulate fumigant in the cabinet by drawing fumigant out of the cabinet (10) into the passageway (28) above the false ceiling (16) and then delivering the fumigant
back into the cabinet during the fumigation stage.

7. An apparatus as claimed in claim 6 characterised
in that the passageway (28) extends along the centre
of the space between the top wall (16) and false
ceiling (14) of the cabinet.

8. An apparatus as claimed in any of claims
5 to 7 in combination with a carrier (26) for matter
to be fumigated to support the matter in the cabinet,
the carrier having a central dividing wall (56) such
that when the carrier (26) is located in the
cabinet with the wall (56) between the two apertures
(23,24) in the false ceiling (16) of the cabinet,
a flow of fumigant down one side of the carrier and
up the other side of the carrier is encouraged.

9. An apparatus as claimed in claim 8
characterised in that a sealing flap extends across
the false ceiling (16) of the cabinet (10) between
the two apertures (23,24) to engage the top of the
carrier (26) and the bottom of the carrier (26) is
supported above the bottom of the cabinet (10)
to encourage the circulation of fumigant down one side of the carrier and up the other side.

10. An apparatus as claimed in claim 9 characterised in that the carrier (26) is mounted on wheels (27) to facilitate its insertion and removal from the cabinet (10) and which also support the bottom of the carrier (26) above the bottom of the cabinet (10) to permit the flow of fumigant under the carrier.

11. An apparatus as claimed in any of the preceding claims characterised in that heating means (35) are provided on the side walls (13) of the cabinet to heat the fumigant flowing past the side walls of the cabinet.

12. An apparatus as claimed in any of the preceding claims characterised in that means (31 to 33) are provided for delivering steam to the cabinet (10) to heat and raise the humidity of the cabinet.

13. An apparatus as claimed in any of the preceding claims characterised in that means (37) are provided for activating a fumigant in the cabinet.

14. An apparatus as claimed in claim 13 characterised in that the means to activate the fumigant in the cabinet (10) comprise means (37) to hold a supply of a fumigant releasing material activated by heat and means to heat the supply of material to cause the release of fumigant.

15. An apparatus as claimed in claim 13 characterised in that the means to activate the fumigant comprise means (37) to hold a supply of one material for producing a fumigant, and means to supply a further material with which the first material reacts to produce fumigant within the cabinet.

16. An apparatus as claimed in any of the preceding claims characterised in that the cabinet (10) has an inlet door (11) one one side and an outlet door (12) on the other side for entry and exit of matter to be fumigated each door having sealing means (22) for sealing the door in the closed position to prevent loss of fumigant during the fumigation operation.

GCB/MCM/EA209

FIG. 1.

FIG. 2.

3/6

FIG. 3.

FIG. 5.

FIG. 4.

FIG. 6.

## FIG. 7

35    34    36

## FIG. 8.

32

31

33

## FIG. 9.

50    52

55

53

FIG. 10.

## EUROPEAN SEARCH REPORT

**European Patent Office**

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| A | GB - A - 1 099 460 (L.W. WOLFE et al.)<br><br>* Whole document * <br><br>-- | 1-16 | A 61 L 2/20<br>A 23 B 5/00<br>A 23 L 3/34<br>A 23 B 7/144<br>      9/00 |
| A | CHEMICAL ABSTRACTS, vol. 87, no. 23, 5th December 1977, page 163, no. 179002y<br>Columbus, Ohio, U.S.A.<br>K. FURUTA et al.: "Studies on the disinfection of hatching eggs. I. The effect of formaldehyde fumigation on bacteria contaminating the egg shell surface"<br><br>& NIPPON KAKIN GAKKAISHI 1977, 14(1), 27-32<br><br>* Abstract *<br><br>---- | | |

**TECHNICAL FIELDS SEARCHED (Int. Cl.³)**

A 61 L 2/20
A 23 L 3/34
A 23 B 5/00
          7/144
          9/00

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 03-03-1981 | MERGONI |

EPO Form 1503.1  06.78